# EUROPÄISCHE PATENTSCHRIFT

(11) **EP 0 555 803 B1**
(45) Veröffentlichungstag und Bekanntmachung des Hinweises auf die Patenterteilung: **08.07.1998**
(21) Anmeldenummer: 93101946.7
(22) Anmeldetag: 08.02.1993
(51) Int. Cl.: A61B 17/32

(54) **Vorrichtung zum Schneiden von biologischem und insbesondere menschlichem Gewebe**
Device for cutting biological tissue especially human tissue
Dispositif pour couper des tissus biologiques spécialement des tissus humains

(30) Priorität: 08.02.1992 DE 4203622
(43) Veröffentlichungstag der Anmeldung: 18.08.1993
(73) Patentinhaber: Karl Storz GmbH & Co., 78532 Tuttlingen (DE)
(72) Erfinder: Steiner, R., Dr. med., CH-8091 Zürich (CH)
(74) Vertreter: Münich, Wilhelm, Dr.

(56) Entgegenhaltungen:
- DE-A- 3 006 577
- DE-A- 3 630 203
- DE-A- 3 828 478
- FR-A- 2 645 008

## Beschreibung

Die Erfindung bezieht sich auf eine Vorrichtung zum Schneiden von biologischem und insbesondere menschlichem Gewebe gemäß dem Oberbegriff des Patentanspruchs 1.

Derartige Vorrichtungen bzw. Instrumente werden beispielsweise bei endoskopischen Eingriffen benötigt, um größere Gewebebereiche abzutrennen, wie es beispielsweise bei der Entnahme von Myomen erforderlich ist.

Eine Vorrichtung, wie sie im Oberbegriff des Patentanspruchs 1 vorausgesetzt wird, ist beispielsweise aus der DE-A-30 06 577 bekannt.

Aus dieser Druckschrift ist eine Vorrichtung zum Schneiden von biologischen und insbesondere von menschlichem Gewebe bekannt, die ein zylindrisches Außenrohr und einen in dem Außenrohr angeordneten Schneid-Hohlzylinder aufweist, den eine proximal in der Längsachse angeordnete Dreheinrichtung zu einer Drehung um seine Längsachse antreibt. Weiterhin weist der Schneid-Hohlzylinder eine Ausnehmung auf, die an einer in etwa parallel zur Längsachse verlaufenden Seite eine Schneidkante aufweist.

Damit ist es bei dem gattungsbildenden Stand der Technik nicht möglich, einen durchgehenden Hohlraum vorzusehen, der gleiche Lumen wie der distal angeordnete Hohlzylinder aufweist. Somit ist es beispielsweise nicht möglich, einen Greifer o. dgl einzusetzen, mit dem das abzutrennende Gewebe gegriffen wird.

Der Erfindung liegt die Aufgabe zugrunde, eine Vorrichtung bzw. ein Instrument zum Schneiden von biologischem und insbesondere menschlichem Gewebe gemäß dem Oberbegriff des Patentanspruchs 1 derart weiterzubilden, ein durchgehender Hohlraum realisiert wird, der das Einsetzen weiterer Instrumente erlaubt, und der vor allem durchgehend das gleiche Lumen aufweist.

Eine erfindungsgemäße Lösung dieser Aufgabe ist im Patentanspruch 1 angegeben. Weiterbildungen der Erfindung sind Gegenstand der Unteransprüche.

Erfindungsgemäß wird eine Vorrichtung bzw. ein Instrument geschaffen, die bzw. das ein zylindrisches Außenrohr und einen in dem Außenrohr angeordneten Schneid-Hohlzylinder aufweist, der gemäß Anspruch 6 insbesondere Bestandteil eines Schneidrohrs sein kann, und der an seiner Vorderseite, mit der er auf das zu schneidende Gewebe aufgesetzt wird, wenigstens eine Schneidkante aufweist. Eine Dreheinrichtung treibt über eine Hohlwelle, die das gleiche Lumen wie der Hohlzylinder freigibt, und die an ihrem proximalen Ende offen ist, den Hohlzylinder zu einer Drehung um seine Längsachse an.

Aufgrund dieser Ausbildung ist es beispielweise möglich, den abzutrennenden Gewebebereich zunächst mit einem Gewebegreifer zu greifen (Anspruch 4), der insbesondere in Art einer herkömmlichen Gewebe-Zange, wie sie handelsüblich von den verschiedensten Herstellern erhältlich ist, ausgebildet sein kann, Anschließend wird der von dem Greifer festgehaltene Gewebebereich durch die Drehung des Schneid-Hohlzylinders, der in Eingriff mit dem Gewebe gebracht wird, aus dem diesen Bereich umgebenden Bereich "herausgeschnitten".

Hierzu weist der Hohlzylinder an seiner Unter- bzw. Vorderseite, mit der er auf das Gewebe aufgesetzt wird, wenigstens eine Schneidkante auf, die glatt oder gezahnt ausgebildet sein kann.

In jedem Falle ist es von Vorteil, wenn der Schneid-Hohlzylinder in dem Außenrohr in Richtung seiner Längsachse verschiebbar ist. Hierdurch ist es möglich, den Schneidvorgang ohne Verschiebung des gesamten Instruments auszuführen. Selbstverständlich ist es aber auch möglich, das Gewebe durch eine Bewegung des das Gewebe greifenden Gewebegreifers hin zum proximalen Ende der Vorrichtung abzutrennen.

Wenn der Schneid-Hohlzylinder hinter das distale Ende des Außenrohrs zurückziehbar bzw. über das distale Ende des Außenrohrs vorschiebbar ist, ist es möglich bei dem Einführen des Instruments die Schneidkante hinter das distale Ende des Außen- bzw. Schutzrohrs zurückzuziehen, so daß eine unbeabsichtigte Verletzung von Gewebe vermieden wird. Ferner kann bei der Entnahme der Schneid-Hohlzylinder mit dem in ihm befindlichen abgetrennten Gewebe in das Außenrohr eingeführt werden, so daß ein unbeabsichtiges Abstreifen des abgetrennten und zu entnehmenden Gewebebereichs nicht möglich ist.

Durch die Anordnung der Dreheinrichtung am proximalen Ende des Instruments, die über eine innerhalb des Außenrohrs verlaufende Hohlwelle den Hohlzylinder antreibt, erhält man ein leichtes und kleines Instrument.

Die Dreheinrichtung kann beispielsweise einen mechanischen oder manuellen Antrieb aufweisen. Da es sich jedoch als vorteilhaft herausgestellt hat, mit Drehzahlen im Bereich von einigen hundert Umdrehungen pro Minute zu arbeiten, ist es besonders bevorzugt, wenn gemäß Anspruch 2 die Dreheinrichtung einen Elektromotor aufweist. Die Achse des Elektromotors schließt dabei bevorzugt einen 90°-Winkel mit der Achse des Instruments ein (Anspruch 3), wobei der Antrieb der des Schneidrohrs am Außenumfang erfolgt. Hierdurch wird das für das Einsetzen des Gewebegreifers verfügbare Lumen nicht durch die Antriebsvorrichtung beeinträchtigt.

Durch die optional mögliche Verschiebbarkeit des Gewebegreifer in Richtung der Längsachse des Außenrohrs ist ein besonders einfaches "Ergreifen" und gegebenenfalls "Heranziehen" des abzutrennenden Gewebebereichs möglich. Hierzu ist es weiter von Vorteil, wenn der Gewebegreifer unabhängig vom Schneid-Hohlzylinder verschiebbar ist. Der Gewebegreifer kann dabei zentrisch oder exzentrisch zu dem Außenrohr angeordnet sein.

Das erfindungsgemäße Instrument ist für die verschiedensten Operationen - sowohl mit Öffnung des Körpers als auch für endoskopische Eingriffe - geeignet. Hierzu ist es von Vorteil, wenn es so ausgebildet ist, daß es in einen herkömmlichen Endoskop-Schaft einsetzbar ist (Anspruch 5). Die Abmessungen des Instruments und insbesondere sein Außendurchmesser können dem jeweiligen Einsatzfall angepaßt sein.

Die Erfindung wird nachstehend anhand eines Ausführungsbeispiels unter Bezugnahme auf die Zeichnung näher beschrieben, auf die im übrigen bezüglich der Offenbarung aller im Text nicht ausdrücklich erwähnten Einzelheiten verwiesen wird. Es zeigen:
- Fig. 1: eine perspektivische Ansicht einer Vorrichtung zum Schneiden von biologischem und insbesondere menschlichem Gewebe, und
- Fig. 2: einen Schnitt längs der Achse des zylindrischen Außenrohrs.

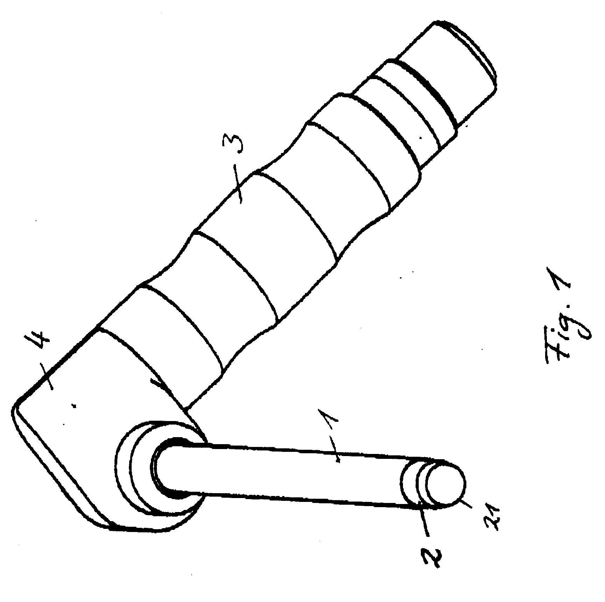

Fig. 1 zeigt in einer perspektivischen Ansicht ein Ausführungsbeispiel einer erfindungsgemäßen Vorrichtung zum Schneiden von biologischem und insbesondere menschlichem Gewebe. Die Vorrichtung weist ein zylindrisches Außenrohr 1 auf, mit dem sie direkt oder in einen nicht dargestellten Endoskop-Schaft eingesetzt beispielsweise in eine Körperhöhle einsetzbar ist. In dem Außenrohr ist ein Schneid-Hohlzylinder bzw. ein Innenrohr 2 drehbar angeordnet, der an seiner Unterseite bzw. an seinem distalen Ende wenigstens eine Schneidkante 21 aufweist.

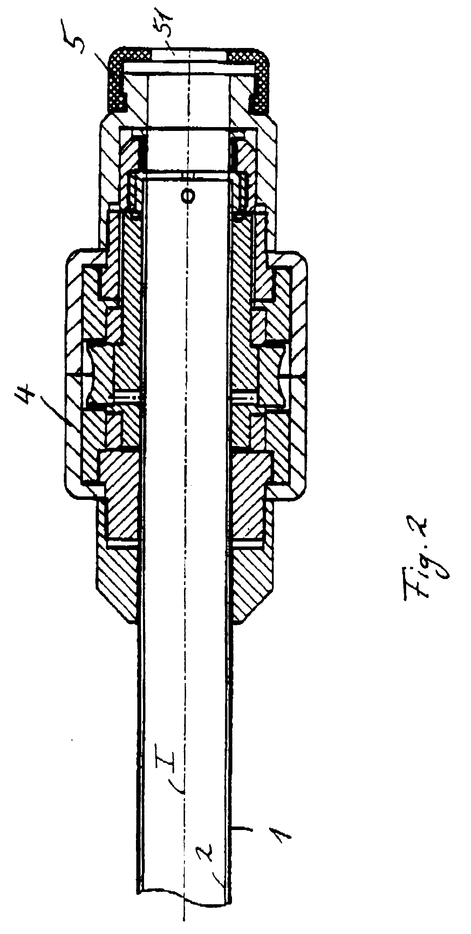

Ferner weist die Vorrichtung einen ergonomisch geformten Handgriff auf, in dem ein nicht gezeigter Elektromotor sowie Batterien bzw. Akkus für diesen Elektromotor untergebracht sind. Der Handgriff ist mit dem aus dem Außenrohr 1 und dem Innenrohr 2 bestehenden eigentlichen Instrument durch einen Verbindungsbereich 4 verbunden, in dem unter anderem das Getriebe untergebracht ist, über das der Elektromotor den Schneid-Hohlzylinder 2 zu einer Drehung um dessen Längsachse (Bezugszeichen I in Fig. 2) antreibt.

Fig. 2 zeigt einen Schnitt durch die in Fig. 1 dargestellte Vorrichtung längs der gemeinsamen Längsachse des Außenrohrs 1 und des Schneid-Hohlzylinders 2. Wie Fig. 2 zu entnehmen ist, weist die Vorrichtung an ihrem proximalen Ende einen Verschlußstopfen 5 auf, der insbesondere aus einem gummiartigen Material bestehen kann, und der eine Öffnung 51 aufweist, die das Einsetzen eines nicht dargestellten Gewebegreifers erlaubt.

Der Gewebegreifer, der insbesondere eine handelsübliche Zange sein kann, ist hierdurch in Richtung der Längsachse I des Außenrohrs 1 unabhängig vom Schneid-Hohlzylinder 2 verschiebbar.

## Patentansprüche

1. Vorrichtung zum Schneiden von biologischem und insbesondere menschlichem Gewebe, mit
- einem zylindrischen Außenrohr (1),
- einem in dem Außenrohr angeordneten Schneid-Hohlzylinder (2), und den eine proximal angeordnete Dreheinrichtung zu einer Drehung um seine Längsachse antreibt,
dadurch **gekennzeichnet**, daß die Dreheinrichtung am Außenumfang einer Hohlwelle angreift, die innerhalb des Außenrohrs verläuft und den Schneid-Hohlzylinder antreibt,
daß der Schneid-Hohlzylinder an seiner Vorderseite, mit der er auf dem zu schneidenden Gewebe aufgesetzt wird, wenigstens eine Schneidkante (21) aufweist, und
daß die Hohlwelle das gleiche Lumen wie der Hohlzylinder freigibt und an ihrem proximalen Ende offen ist.

2. Vorrichtung nach Anspruch 1,
dadurch **gekennzeichnet**, daß die Dreheinrichtung einen Elektromotor aufweist.

3. Vorrichtung nach Anspruch 2,
dadurch **gekennzeichnet**, daß die Achse des Elektromotors einen 90°-Winkel mit der Achse der Welle bzw. des Hohlzylinders einschließt.

4. Vorrichtung nach einem der Ansprüche 1 bis 3,
dadurch **gekennzeichnet**, daß in dem Schneid-Hohlzylinder und der Hohlwelle ein Gewebegreifer angeordnet ist.

5. Vorrichtung nach einem der Ansprüche 1 bis 4,
dadurch **gekennzeichnet**, daß sie in einen Endoskop-Schaft einsetzbar ist.

6. Vorrichtung nach einem der Ansprüche 1 bis 5,
dadurch **gekennzeichnet**, daß ein Schneidrohr den Schneid-Hohlzylinder und die Hohlwelle bildet.

## Claims

1. An device for cutting biological and, in particular, human tissue having
- a cylindrical external tube (1),
- a cutting hollow cylinder (2) disposed in said external tube, and which a proximally disposed rotation device drives to revolve about its longitudinal axis,
**characterized by** the fact that said rotation device engages at the external circumference of a hollow shaft which runs inside said external tube and drives said cutting hollow cylinder,
that said cutting hollow cylinder is provided with at least one cutting edge (21) at its front side with which it is placed on the to-be-cut tissue, and
that said hollow shaft releases the same lumen as said hollow cylinder and is open at its proximal end.

2. A device according to claim 1,
**characterized by** the fact that said rotation device is provided with an electric engine.

3. A device according to claim 2,
**characterized by** the fact that the axis of said electric engine forms a 90° angle with the axis of said shaft respectively of said hollow cylinder.

4. A device according to one of the claims 1 to 3,
**characterized by** the fact that a tissue gripper is disposed in said cutting hollow cylinder and said hollow shaft.

5. A device according to one of the claims 1 to 4,
**characterized by** the fact that said device can be inserted in an endoscope.

6. A device according to one of the claims 1 to 5,
**characterized by** the fact that a cutting tube forms said cutting hollow cylinder and said hollow shaft.

## Revendications

1. Dispositif pour couper un tissu biologique et notamment un tissu humain, comportant
- un tube extérieur cylindrique (1) ;
- un cylindre creux de coupe (2), qui est disposé dans le tube extérieur et qu'un dispositif d'entraînement en rotation, disposé sur le côté proximal, entraîne en rotation autour de son axe longitudinal, caractérisé en ce
que le dispositif d'entraînement en rotation attaque la périphérie extérieure d'un arbre creux, qui est disposé à l'intérieur d'un tube extérieur et entraîne le cylindre creux de coupe, et
que le cylindre creux de coupe comporte au moins une arête de coupe (21) sur son côté avant, avec lequel il est appliqué contre le tissu à couper, et
que l'arbre creux libère le même passage intérieur que le cylindre creux et est ouvert au niveau de son extrémité proximale.

2. Dispositif selon la revendication 1, caractérisé en ce que le dispositif d'entraînement en rotation possède un moteur électrique.

3. Dispositif selon la revendications 2, caractérisé en ce que l'axe du moteur électrique fait un angle de 90° avec l'axe de l'arbre ou du cylindre creux.

4. Dispositif selon l'une des revendications 1 à 3, caractérisé en ce qu'un organe de saisie du tissu est disposé dans le cylindre creux de coupe et dans l'arbre creux.

5. Dispositif selon l'une des revendications 1 à 4, caractérisé en ce qu'il peut être inséré dans une tige d'endoscope.

6. Dispositif selon l'une des revendications 1 à 5, caractérisé en ce qu'un tube de coupe forme le cylindre creux de coupe et l'arbre creux.
